# EUROPEAN PATENT SPECIFICATION

(11) **EP 0 975 272 B1**
(45) Date of publication and mention of the grant of the patent: **29.06.2005**
(21) Application number: 98901454.3
(22) Date of filing: 16.02.1998
(51) Int. Cl.: A61B 19/00

(54) **IMAGE-GUIDED SURGERY SYSTEM**
CHIRURGISCHES SYSTEM MIT BILDFÜHRUNG
SYSTEME DE CHIRURGIE GUIDE PAR L'IMAGE

(30) Priority: 28.02.1997 EP 97200600
(43) Date of publication of application: 02.02.2000
(73) Proprietor: Koninklijke Philips Electronics N.V., 5621 BA Eindhoven (NL)
(72) Inventor: VAN DER BRUG, Willem, Peter, NL-5656 AA Eindhoven (NL); DE BLIEK, Hubrecht, Lambertus, Tjalling, NL-5656 AA Eindhoven (NL); GERRITSEN, Frans, Andreas, NL-5656 AA Eindhoven (NL)
(74) Representative: Cohen, Julius Simon
(86) International application number: PCT/IB1998/000190
(87) International publication number: WO 1998/037827

(56) References cited:
- EP-A- 0 676 178
- EP-A- 0 755 660

## Description

The invention relates to an image-guided surgery system. An image-guided surgery system according to the preamble of claim 1 is disclosed in EP 0 755 660 A.

Another image-guided surgery system of this kind is known from United States patent specification US 5,389,101.

An image-guided surgery system is used to visualize a position of a surgical instrument in an operating area within the body of a patient for a surgeon during surgery. Images, such as CT or MRI images, are made of the patient prior to surgery. The image-guided surgery system includes a position-measuring system for measuring the position of the surgical instrument. The image-guided surgery system also includes a computer for deriving corresponding positions in a relevant image from the positions of the surgical instrument measured. During surgery the position measuring system measures the position of the surgical instrument relative to the patient and the computer calculates the position in such a prior image which corresponds to the measured position of the surgical instrument. The prior image is then displayed on a monitor, together with the actual position of the surgical instrument. The surgeon can see the position of the surgical instrument in the operating area in the image on the monitor, without him or her seeing the surgical instrument directly. The surgeon can thus observe the image displayed on the monitor so as to see how to move the surgical instrument in the operating area without substantial risk of unnecessary damaging of tissue, and notably without risk of damaging of vital parts.

An image-guided surgery system of this kind is used, for example in neurosurgery in order to show the surgeon accurately where the surgical instrument is situated in the brain during a cerebral operation.

Using the known image-guided surgery system it is difficult to position the instrument with one end in a desired position. In order to move the instrument to the desired position, it is necessary to move it whereas at the same time the image showing the position of the surgical instrument relative to the patient must be observed so as to determine whether the desired position has been reached. It has been found that accurate positioning of the instrument requires a substantial amount of training and that these operations remain time consuming still.

It is an object of the invention to provide an image-guided surgery system in which accurate positioning of the surgical instrument is easier and faster.

This object is achieved by means of an image-guided surgery system according to the invention which includes an alignment system for deriving an alignment line through a target position, for selecting a starting plane through an end of a surgical instrument, for deriving a starting point on the alignment line and in the starting plane, and for reproducing a position of said end and of said starting point relative to one another in the starting plane.

The target position is a position which is usually situated in or on the body of the patient and whereto the end of the surgical instrument is to be guided during a given phase of the operation. During the operation, the surgical instrument is introduced into the body of the patient via an entrance position on the surface. The target position may in some cases be the final position to be ultimately reached by the end of the surgical instrument. At the final position there is situated, for example a tumor to be removed. More generally speaking, however, the target position is situated somewhere between the final position and the entrance position, for example, when the final position is to be reached along a curved path extending from the entrance position. The alignment line passes through the target position and has a direction via which the target position can be readily approached. For example, the alignment line extends through the entrance position and the target position. The user preferably chooses the entrance position so that the target position can be readily reached from the entrance position. At the beginning of the surgical intervention the surgical instrument is positioned at the starting point. This can be very readily achieved by moving the end of a surgical instrument in the starting plane until this end reaches the starting point. The alignment system shows the user how to move the end to the starting point. To this end, the alignment system shows, for example the positions of the end and the starting point in the starting plane. The positions of the starting point and the end of the surgical instrument can be revealed to the user in various ways. For example, it is very simple to state the co-ordinates of the starting point and the end of the surgical instrument in the starting plane. It is particularly attractive to display a guide image on an image display unit such as a monitor. The guide image is an image of the starting plane with images of the starting point and the end of the surgical instrument. The user can observe the guide image so as to see how the end of the surgical instrument moves in the starting plane so that the user knows how to move the end of the surgical instrument to the starting point. Another possibility is that the alignment system shows the direction in which the end of the surgical instrument must be moved in order to reach the starting point, for example by indicating the desired direction by means of arrows. The user need not take into account displacement of the end of the surgical instrument parallel to the alignment line. Thus, it is much easier to move the end of the surgical instrument in a direction which is limited to one plane, i.e. the starting plane, than to move the end through the three-dimensional space. The starting plane always contains the end of the surgical instrument. The starting plane can be selected by selecting an auxiliary plane which is situated a predetermined, fixed distance from the end of the surgical instrument. The starting plane is then the plane extending parallel to the auxiliary plane at the predetermined, fixed distance from the auxiliary plane. For example, the auxiliary plane can be chosen so as to extend through a predetermined point on the surgical instrument which is situated at the predetermined, fixed distance from the end of the surgical instrument.

The starting plane moves in space in dependence on the movement of the surgical instrument. When the starting plane changes relative to the patient, the position of the starting point relative to the patient also changes, but for practically all surgical interventions it is not important where on the alignment line the starting point is situated. The alignment system reproduces the instantaneous position of the end of the surgical instrument and the position of the starting point. This enables the user to move the surgical instrument to the starting position quickly and accurately in a controlled manner.

The surgical instrument is, for example an instrument used to perform the relevant surgical intervention. The surgical instrument may also be a pointer which is positioned in the starting position and is subsequently replaced by the instrument whereby the surgical intervention is performed.

The image-guided surgery system according to the invention is suitable for use for a variety of surgical interventions, notably for neurosurgery such as cerebral operations and spinal operations.

The alignment system in a preferred embodiment of an image-guided surgery system according to the invention is arranged to reproduce the starting point substantially at the center of the guide image.

Because the starting point is reproduced substantially at the center of the guide image, the user can see very well how the surgical instrument must be moved so as to move the end to the starting point. Furthermore, the surgical instrument is hardly ever moved so that the reproduction of the end of the surgical instrument is moved (almost) out of the guide image, because the end of the surgical instrument is reproduced approximately at the center of the guide image during its displacement to the starting point. Moreover, there is not much risk of confusion between the starting point and the position of the end of the surgical instrument, because it is decided in advance that the starting point will be shown at the center of the guide image.

The alignment system in a preferred embodiment of an image-guided surgery system according to the invention is arranged to select the starting plane so as to extend substantially perpendicularly to the alignment line.

Consequently, the starting point will not be situated too far from the position of the end of the surgical instrument when the user starts to move the surgical instrument to the starting point, so that the surgical instrument need be displaced over a short distance only. Consequently, only a small amount of time is required to move the end of the surgical instrument accurately to the starting point. Moreover, it can be readily ensured that the starting point is not situated too far from the patient, so that the surgical instrument can be readily moved accurately to the target position along the alignment line.

The image display system in a preferred embodiment of an image-guided surgery system according to the invention is arranged to adjust the ratio of the distance between said end and the starting point to the distance between the reproduction of the end and the reproduction of the starting point in the guide image on the basis of the distance between said end and the starting point.

Via adjustment of the ratio, the guide image is reproduced with a magnification or reduction which is dependent on the distance between the end of the surgical instrument and the starting point. Preferably, a small magnification or even a reduction is used for as long as the end of the surgical instrument is still remote from the starting point, a (larger) magnification being used when the end of the surgical instrument reaches the vicinity of the starting point. The user can thus always see very well how the end of the surgical instrument must be displaced to the starting point because, for as long as the end of the surgical instrument is still remote from the starting point, the reproduction of the end will be situated within the guide image and the reproductions of the end of the surgical instrument and of the starting point can both be observed in the guide image. As soon as the end of the surgical instrument reaches the vicinity of the starting point, they will both be suitably separately reproduced in the guide image. Preferably, the reproduction of the guide image is step-wise enlarged as the end of the surgical instrument approaches the starting point.

The alignment system in a preferred embodiment of an image-guided surgery system according to the invention includes a rotatable alignment member for supporting the surgical instrument, the end of the surgical instrument being retained in a fixed position in the alignment member which is arranged to lock the end of the surgical element in the starting point, the alignment system being arranged to select the starting plane so as to extend through said fixed position in the alignment member.

The starting plane through the fixed position in the alignment member contains the end of the surgical instrument, because this end is retained in said fixed position relative to the alignment member. When the alignment member locks the end of the surgical instrument in the starting point, rotation of the surgical instrument about the end is still possible. The surgical instrument is oriented in the direction of the alignment line by rotating the alignment member supporting the surgical element.

The alignment system in a preferred embodiment of an image-guided surgery system according to the invention is arranged to reproduce a distance between the end of the surgical instrument and the target position.

The surgical instrument can thus be easily moved in the direction of the alignment line until the target position is reached. This is attractive notably when a biopsy is performed. The surgical instrument then consists of a biopsy needle which is positioned in the target position where it takes a sample of the tissue. The alignment system in a preferred embodiment of an image-guided surgery system according to the invention includes an instrument holder which is arranged to adjust the distance between the end of the surgical instrument and the target position while maintaining the orientation of the surgical instrument in the direction of the alignment line.

Such an instrument holder supports the surgical instrument. It enables accurate and easy displacement of the surgical instrument in the direction of the alignment line so as to reach the target position without excessive risk to the patient.

The invention also relates to a method of aligning a surgical instrument as defined in Claim 10.

The method according to the invention achieves easier, faster and more accurate alignment of the surgical instrument, as compared to the alignment performed with the known image-guided surgery system.

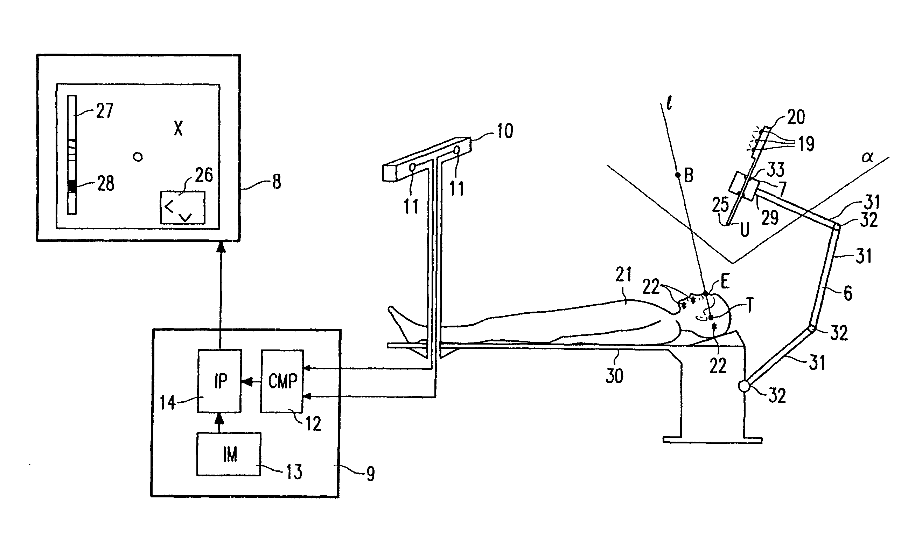

These and other aspects of the invention will be illustrated with reference to the following embodiments and the accompanying drawing; the Figure shows diagrammatically an image-guided surgery system in which the invention is used.

The image-guided surgery system includes a position measuring system with a camera unit 10 and two CCD image sensors 11. The camera unit 10 is mounted on a patient table 30. The camera unit 10 forms images of the surgical instrument 20 from different directions. The surgical instrument is provided with a plurality of, for example three infrared emitting diodes (IREDs) 19. The CCD image sensors produce image signals, notably electronic video signals, which represent the individual images of the surgical instrument 20, notably of the IREDs 19. The position measuring system also includes a computer 12 for deriving the position of the surgical instrument from the image signals. Image information of the patient 21 to be examined or treated is stored in an image memory 13. The image information concerns, for example MRI and/or CT images formed before or during the surgical treatment. Marks 23 notably formed by fiducial markers, on or in the patient 21 are also reproduced in the images of the patient. The positions of the marks 22 are measured by means of the position measuring system, for example by pointing out the marks by means of the surgical instrument. The computer 12 derives the relation between positions in or on the patient 21 and the corresponding positions in the images from the positions of the marks and the positions of the images of the marks in the images formed. On the basis of the measured position of the surgical instrument 20 and said relation, the image processor 14 forms an image signal which represents an image which shows image information of the patient 21 and also the instantaneous position of the surgical instrument 20 in the patient. The computer 12, the image memory 13 and the image processor are included in a data processor 9 whereto a monitor 8 is connected. The image signal is applied to the monitor 8. Image information of the patient 21, showing where the surgical instrument 20 is situated, is displayed on the monitor 8. The user can thus move the surgical instrument 20 within the patient 21 without having a direct view of the instrument and without risk of unnecessary damaging of tissue.

The image-guided surgery system according to the invention includes an alignment system by means of which the user can readily position the surgical instrument 20 in a suitable starting position B. The alignment system includes the camera unit 10, the computer 12, the image processor 14 and the monitor 8. Preferably, the alignment system also includes a system of arms 6 which supports the alignment member 7. Prior to the surgical intervention, for example a biopsy, the user selects the target position T and also the alignment line 1 from the pre-recorded image information. The alignment line 1 passes through the target position T and through the entrance position E. The entrance position E is selected to be such that the target position T can be readily reached along the alignment line, without unnecessary damaging of tissues or without excessive risk to the patient. The position measuring system 10, 12 measures the position of the end U of the surgical instrument. The computer 12 calculates the position of the target position T and the entrance position E from the positions of the reproductions of the target and entrance position selected in the image information by the user. The computer 12 subsequently calculates the starting plane α which extends through the instantaneous position U of the end 25 of the surgical instrument and preferably perpendicularly to the alignment line 1. The computer 12 also calculates the point of intersection between the alignment line 1 and the starting plane α; this point of intersection is the starting point B. The computer 12 then calculates the positions of the end U and the starting point B in the starting plane α. On the basis of these positions, the image processor forms an image signal which represents the guide image. Such a guide image is displayed on the monitor 18 and shows the position U of the end 25 and the position of the starting point B in the starting plane α. In the example shown in the Figure, the position of the starting point is shown at the center of the image, denoted by a circle, and the position of the end 25 is denoted by a cross. The user can readily move the end 25 of the surgical instrument 20 to the starting position B while observing the guide image. Adjacent to or instead of the position of the starting position B and the position U of the end 25, the image processor 14 can provide arrows 26 in the guide image on the monitor 8 so as to indicate the directions in which the end 25 must be moved in the starting plane α so as to reach the starting point B. It is not essential that the end 25 of the surgical instrument is moved parallel to the direction of the alignment line during its displacement to the starting position B by the user. The computer automatically adapts the position of the starting plane α by ensuring that the starting plane α always extends through the position U of the end 25. The starting point is automatically adapted to the movement of the starting plane α because the starting point is the point of intersection between the alignment line 1 and the starting plane α. It has been found that it is not very important where exactly on the alignment line 1 the starting point B is situated.

The computer 12 also calculates the distance between the end 25 of the surgical instrument and the target position T; this calculation preferably takes place when the end 25 has been placed in the starting position B. On the basis of this distance the image processor 14 controls the monitor 8 so as to provide a depth indication. The depth indication is, for example a scale graduation 27 on which the distance between the end 25 and the target position T is indicated by means of a pointer 28. The user can thus see on the monitor how far the end 25 must be moved in the direction of the alignment line so as to reach the target position T.

The alignment system also includes a system of arms 6 with a plurality of arms 31 which are rotatably coupled to one another, for example by means of hinges 32. The alignment member 7 is secured to an end 29 of the system of arms. The hinges can be locked so as to retain the alignment member 7 in a desired position. The surgical instrument 20 fits in an opening 33 in the alignment member 7. The alignment member 7 is, for example a sphere in which a cylindrical channel 33 is recessed so as to extend through the center of the sphere. Using the alignment member 7, the surgical instrument 20 can be readily aligned in the direction of the alignment line 1, the end being retained in the starting position B. By sliding the surgical instrument 20 through the channel 33, the end 25 is moved accurately in the direction of the alignment line 1 to the target position T. When such an alignment member is used, the depth indication can be simply used to arrange the alignment member at a fixed distance from the target position T. Such a fixed distance is, for example a standard length of the biopsy needle. When the alignment member has been arranged at the desired, fixed distance from the target position on the basis of the depth indication, the biopsy needle moved into the patient via the alignment member will accurately reach the target position.

## Claims

1. Image-guided surgery system comprising
- means (6, 7, 31, 32) for holding and displacing a surgical instrument (20), the surgical instrument having an end (25) arranged to perform a surgical operation,
- means (10, 11, 12, 19) for measuring the position of the surgical instrument in relation to a patient (21) to be treated,
- display means (8) for displaying the position of the end (25) of the surgical instrument in relation to a preselected alignment line (1),
**characterized in that**
- means (12, 14) are provided for
- selecting a starting plane (α), said starting plane (α) extending through the instantaneous position (U) of the end (25) of the surgical instrument and including one point of the preselected alignment line,
- calculating a starting point (B) on the preselected alignment line (1), the starting point (B) being the point of intersection between the preselected alignment line and the a starting plane (α),
- reproducing a guide image showing the position of the starting point (B) and the instantaneous position (U) of the end (25) of the surgical instrument in the starting plane (α) via the display means (8).

2. Image-guided surgery system according to Claim 1, **characterized in that**
- the means (12) for calculating the starting point (B) are arranged to move the starting plane (α) together with a displacement of the end (25) of the surgical instrument.

3. Image-guided surgery system according to Claim 1 or 2, **characterized in that**
- the means for measuring the position of the surgical instrument in relation to a patient (21) to be treated comprise
- a camera unit (10) having two image sensors (11) and
- a plurality of infrared emitting diodes (19) being provided on the surgical instrument (20).

4. Image-guided surgery system according to one of the Claims 1 to 3, **characterized in that**,
- the means for reproducing a guide image showing the position of the starting point (B) and the instantaneous position (U) of the end (25) of the surgical instrument in the starting plane (α) via the display means (8) are arranged to reproduce the starting point (B) substantially at the centre of the guide image.

5. Image-guided surgery according to one of the Claims 1 to 4, **characterized in that**
- the means for reproducing a guide image showing the position of the starting point (B) and the instantaneous position (U) of the end (25) of the surgical instrument in the starting plane (α) via the display means (8) are arranged to adjust the ratio of the distance between said end (25) and the starting point (B) to the distance between the reproduction of the said end (25) and the reproduction (B) of the starting point in the guide image on the basis of the distance between said end and the starting point.

6. Image-guided surgery system according to one of the Claims 1 to 5, **characterized in that**
- the means (12) for calculating a starting point (B) are arranged to select the starting plane (α) so as to extend substantially perpendicularly to the alignment line (1).

7. Image-guided surgery system according to one of the Claims 1 to 6, **characterized in that**
- the means for holding and displacing a surgical instrument (20) comprise a rotatable alignment member (7) for supporting the surgical instrument, the end (25) of the surgical instrument being retained in a fixed position in relation to the alignment member, and
- the alignment member (7) being arranged to lock the end (25) of the surgical element in the starting point (B).

8. Image-guided surgery system according to one of the Claims 1 to 7, **characterized in that**
- the means (12, 14) for reproducing a guide image via the display means (8) are arranged to reproduce the distance between the end (25) of the surgical instrument and a target position (T).

9. Image-guided surgery system according to one of the Claims 1 to 8, **characterized in that**
- the means for holding and displacing a surgical instrument comprise an instrument holder which is arranged to adjust the distance between the end (25) of the surgical instrument and the target position while maintaining the orientation of the surgical instrument in the direction of the alignment line.

## Patentansprüche

1. Bildgeführtes Operationssystem, mit
- Mitteln (6, 7, 31, 32) zum Halten und Verlagern eines chirurgischen Instruments (20), wobei das chirurgische Instrument ein zum Ausführen einer chirurgischen Operation ausgebildetes Ende (25) aufweist,
- Mitteln (10, 11, 12, 19) zum Messen der Position des chirurgischen Instruments in Relation zu einem zu behandelnden Patienten (21),
- Anzeigemitteln (8) zum Anzeigen der Position des Endes (25) des chirurgischen Instruments in Relation zu einer zuvor gewählten Ausrichtlinie (1),
**dadurch gekennzeichnet, dass**
- Mittel (12, 14) vorgesehen sind zum
- Wählen einer Anfangsebene (α), welche Anfangsebene (α), durch die momentane Position (U) des Endes (25) des chirurgischen Instruments verläuft und einen Punkt der zuvor gewählten Ausrichtlinie enthält,
- Berechnen eines Anfangspunktes (B) auf der zuvor gewählten Ausrichtlinie (1), wobei der Anfangspunkt (B) der Schnittpunkt zwischen der zuvor gewählten Ausrichtlinie und der Anfangsebene(α) ist,
- Wiedergeben, mittels der Anzeigemittel (8), eines Führungsbildes, das die Position des Anfangspunktes (B) und die momentane Position (U) des Endes (25) des chirurgischen Instruments in der Anfangsebene(α) zeigt.

2. Bildgeführtes Operationssystem nach Anspruch 1, **dadurch gekennzeichnet, dass**
- die Mittel (12) zum Berechnen des Anfangspunktes (B) ausgebildet sind, um die Anfangsebene (α) zusammen mit einer Verlagerung des Endes (25) des chirurgischen Instruments zu bewegen.

3. Bildgeführtes Operationssystem nach Anspruch 1 oder 2, **dadurch gekennzeichnet, dass**
- die Mittel zum Messen der Position des chirurgischen Instruments in Relation zu einem zu behandelnden Patienten (21)
- eine Kameraeinheit (10) mit zwei Bildsensoren (11) umfassen und
- eine Vielzahl infrarot emittierender Dioden (19) auf dem chirurgischen Instrument (20) angebracht ist.

4. Bildgeführtes Operationssystem nach einem der Ansprüche 1 bis 3, **dadurch gekennzeichnet, dass**
- die Mittel zum Wiedergeben, mittels der Anzeigemittel (8), eines Führungsbildes, das die Position des Anfangspunktes (B) und die momentane Position (U) des Endes (25) des chirurgischen Instruments in der Anfangsebene (α) zeigt, ausgebildet sind, um den Anfangspunkt (B) nahezu in der Mitte des Führungsbildes wiederzugeben.

5. Bildgeführtes Operationssystem nach einem der Ansprüche 1 bis 4, **dadurch gekennzeichnet, dass**
- die Mittel zum Wiedergeben, mittels der Anzeigemittel (8), eines Führungsbildes, das die Position des Anfangspunktes (B) und die momentane Position (U) des Endes (25) des chirurgischen Instruments in der Anfangsebene (α) zeigt, ausgebildet sind, um das Verhältnis des Abstandes zwischen dem genannten Ende (25) und dem Anfangspunkt (B) zum Abstand zwischen der Wiedergabe des genannten Endes (25) und der Wiedergabe des Anfangspunktes (B) in dem Führungsbild auf Basis des Abstandes zwischen dem genannten Ende und dem Anfangspunkt einzustellen.

6. Bildgeführtes Operationssystem nach einem der Ansprüche 1 bis 5, **dadurch gekennzeichnet, dass**
- die Mittel (12) zum Berechnen eines Anfangspunktes (B) ausgebildet sind, um die Anfangsebene (α) so zu wählen, dass sie nahezu senkrecht zu der Ausrichtlinie (1) verläuft.

7. Bildgeführtes Operationssystem nach einem der Ansprüche 1 bis 6, **dadurch gekennzeichnet, dass**
- die Mittel zum Halten und Verlagern eines chirurgischen Instruments (20) ein drehbares Ausrichtglied (7) zum Tragen des chirurgischen Instruments umfassen, wobei das Ende (25) des chirurgischen Instruments in Relation zu dem Ausrichtglied in einer festen Position gehalten wird, und
- das Ausrichtglied (7) ausgebildet ist, um das Ende (25) des chirurgischen Elements im Anfangspunkt (B) zu verriegeln.

8. Bildgeführtes Operationssystem nach einem der Ansprüche 1 bis 7, **dadurch gekennzeichnet, dass**
- die Mittel (12, 14) zum Wiedergeben eines Führungsbildes mittels der Anzeigemittel (8) ausgebildet sind, um den Abstand zwischen dem Ende (25) des chirurgischen Instruments und einer Zielposition (T) wiederzugeben.

9. Bildgeführtes Operationssystem nach einem der Ansprüche 1 bis 8, **dadurch gekennzeichnet, dass**
- die Mittel zum Halten und Verlagern eines chirurgischen Instruments einen Instrumenthalter umfassen, der ausgebildet ist, um den Abstand zwischen dem Ende (25) des chirurgischen Instruments und der Zielposition einzustellen, wobei die Orientierung des chirurgischen Instruments in Richtung der Ausrichtlinie erhalten bleibt.

## Revendications

1. Système de chirurgie guidée par l'image comprenant
- des moyens (6, 7, 31, 32) pour tenir et déplacer un instrument chirurgical (20), l'instrument chirurgical présentant une extrémité (25) à même d'exécuter une opération chirurgicale,
- des moyens (10, 11, 12, 19) pour mesurer la position de l'instrument chirurgical par rapport à un patient (21) devant être traité,
- un moyen d'affichage (8) pour afficher la position de l'extrémité (25) de l'instrument chirurgical par rapport à une ligne d'alignement présélectionnée (1),
**caractérisé en ce que**
- des moyens (12, 14) sont prévus pour
- sélectionner un plan de départ (α), ledit plan de départ (α) passant par la position instantanée (U) de l'extrémité (25) de l'instrument chirurgical et incluant un point de la ligne d'alignement présélectionnée,
- calculer un point de départ (B) sur la ligne d'alignement présélectionnée (1), le point de départ (B) étant le point d'intersection entre la ligne d'alignement présélectionnée et le plan de départ (α),
- reproduire une image guide montrant la position du point de départ (B) et la position instantanée (U) de l'extrémité (25) de l'instrument chirurgical dans le plan de départ (α) par le biais du moyen d'affichage (8).

2. Système de chirurgie guidée par l'image selon la revendication 1, **caractérisé en ce que**
- le moyen (12) pour calculer le point de départ (B) est à même de déplacer le plan de départ (α) en même temps qu'un déplacement de l'extrémité (25) de l'instrument chirurgical.

3. Système de chirurgie guidée par l'image selon la revendication 1 ou 2, **caractérisé en ce que**
- les moyens pour mesurer la position de l'instrument chirurgical par rapport à un patient (21) devant être traité comprennent
- une unité de caméra (10) présentant deux capteurs d'images (11) et
- une pluralité de diodes infrarouges (19) prévues sur l'instrument chirurgical (20).

4. Système de chirurgie guidée par l'image selon l'une des revendications 1 à 3, **caractérisé en ce que**
- les moyens pour reproduire une image guide montrant la position du point de départ (B) et la position instantanée (U) de l'extrémité (25) de l'instrument chirurgical dans le plan de départ (α) par le biais du moyen d'affichage (8) sont à même de reproduire le point de départ (B) substantiellement au centre de l'image guide.

5. Système de chirurgie guidée par l'image selon l'une des revendications 1 à 4, **caractérisé en ce que**
- les moyens pour reproduire une image guide montrant la position du point de départ (B) et la position instantanée (U) de l'extrémité (25) de l'instrument chirurgical dans le plan de départ (α) par le biais du moyen d'affichage (8) sont à même d'ajuster le rapport de la distance entre ladite extrémité (25) et le point de départ (B) à la distance entre la reproduction de ladite extrémité (25) et la reproduction (B) du point de départ dans l'image guide sur la base de la distance entre ladite extrémité et le point de départ.

6. Système de chirurgie guidée par l'image selon l'une des revendications 1 à 5, **caractérisé en ce que**
- le moyen (12) pour calculer un point de départ (B) est à même de sélectionner le plan de départ (α) de manière à ce qu'il soit substantiellement perpendiculaire à la ligne d'alignement (1).

7. Système de chirurgie guidée par l'image selon l'une des revendications 1 à 6, **caractérisé en ce que**
- les moyens pour tenir et déplacer un instrument chirurgical (20) comprennent un organe d'alignement rotatif (7) pour supporter l'instrument chirurgical, l'extrémité (25) de l'instrument chirurgical étant retenue dans une position fixe par rapport à l'organe d'alignement, et
- l'organe d'alignement (7) est à même de verrouiller l'extrémité (25) de l'instrument chirurgical au niveau du point de départ (B).

8. Système de chirurgie guidée par l'image selon l'une des revendications 1 à 7, **caractérisé en ce que**
- les moyens (12, 14) pour reproduire une image guide par le biais du moyen d'affichage (8) sont à même de reproduire la distance entre l'extrémité (25) de l'instrument chirurgical et une position cible (T).

9. Système de chirurgie guidée par l'image selon l'une des revendications 1 à 8, **caractérisé en ce que**
- les moyens pour tenir et déplacer un instrument chirurgical comprennent un support d'instrument qui est à même d'ajuster la distance entre l'extrémité (25) de l'instrument chirurgical et la position cible tout en maintenant l'orientation de l'instrument chirurgical dans la direction de la ligne d'alignement.
